# EUROPEAN PATENT SPECIFICATION

(11) **EP 4 472 489 B1**
(45) Date of publication and mention of the grant of the patent: **28.05.2025**
(21) Application number: 23702557.2
(22) Date of filing: 29.01.2023
(51) Int. Cl.: A61B 5/00, A61B 5/28, A61B 5/301, H04B 10/516, H04B 10/80

(54) **SYSTEM FOR MONITORING ELECTROCARDIOGRAM PULSES USING VIRTUAL GROUND**
SYSTEM ZUR ÜBERWACHUNG VON ELEKTROKARDIOGRAMMIMPULSEN MITTELS VIRTUELLER MASSE
SYSTÈME DE SURVEILLANCE D'IMPULSIONS D'ÉLECTROCARDIOGRAMME UTILISANT UNE MASSE VIRTUELLE

(30) Priority: 02.02.2022 US 202263305800 P
(43) Date of publication of application: 11.12.2024
(73) Proprietor: Koninklijke Philips N.V., 5656 AG Eindhoven (NL)
(72) Inventor: BROWN, Richard Earl, 5656 AG Eindhoven (NL); REDDER, Paul Franz, 5656 AG Eindhoven (NL); APPLETON, Bruce Geoffrey, 5656 AG Eindhoven (NL)
(74) Representative: Philips Intellectual Property & Standards
(86) International application number: PCT/EP2023/052104
(87) International publication number: WO 2023/148112

(56) References cited:
- EP-B1- 3 422 607
- JP-A- 2007 222 475
- KR-B1- 101 440 458
- US-A- 6 117 076
- US-A1- 2017 052 559
- US-A1- 2019 282 817

## Description

### BACKGROUND

Electrocardiogram (ECG) systems monitor functionality of a subject's heart by acquiring and measuring ECG pulses though ECG electrodes placed in contact with the subject. ECG systems may be useful in monitoring subjects in potentially stressful situations during medical diagnostics and treatment. For example, during a magnetic resonance imaging (MRI) procedure, the subject is confined to a relatively small diameter bore of an MRI scanner for an extended period of time, which may cause anxiety. Therefore, ECG electrodes may be attached to the subject while inside the bore during the MRI procedure to provide ECG pulses in real-time, and thus information regarding the subject's well-being.

However, the MRI scanner is a harsh environment for detecting small, millivolt, ECG pulses produced by the heart. Conventional ECG systems are implemented with long electrical ECG leads individually connecting the ECG electrodes to an ECG module, which serves as the analog front end for the ECG electrodes, including amplification and analog-to-digital conversion of the ECG pulses. The ECG leads are susceptible to MRI noise pickup during active scans resulting in ECG signal degradation. The ECG leads may also be a source of radio frequency (RF) heating, induced by RF coils of the MRI scanner, which may cause thermal injuries to the subject if the ECG leads are not placed correctly. JP2007222475A relates to an electrocardiographic measurement apparatus and an MRI imaging system for electrocardiographic MRI that detects an electrocardiographic signal in an MRI apparatus and performs imaging in synchronization with the motion of a subject's heart.

### SUMMARY

The present invention is defined by independent claims 1 and 14. The dependent claims define further embodiments of the invention.

According to a representative embodiment, a system is provided for acquiring electrocardiogram (ECG) pulses from a subject. The system includes a common node configured to create a virtual ground, and multiple measurement nodes connectable to corresponding ECG electrodes, respectively, attachable to the subject. The measurement nodes are connected to the virtual ground at the common node via short conductive paths, respectively. Each measurement node of the multiple measurement nodes includes an analog-to-digital converter (ADC) configured to convert an ECG signal from the corresponding ECG electrode to a digital signal; an optical converter configured to convert the digital signal from the ADC to an optical signal, and to output the optical signal via an output fiber-optic cable; a direct current (DC) power converter configured to receive a modulated optical signal with an embedded clock signal via an input fiber-optic cable, to recover DC power from the modulated optical signal, and to supply the DC power to at least the ADC and the optical converter; and a clock recovery circuit configured to receive the modulated optical signal with the embedded clock signal via the input fiber-optic cable, to recover the embedded clock signal from the modulated optical signal, and to supply the recovered clock signal to at least the ADC and the optical converter for synchronization. The common node may or may not be configured as a measurement node.

According to another representative embodiment, a system is provided for acquiring ECG pulses from a subject. The system includes a common node configured to create a virtual ground, and multiple measurement nodes in contact with corresponding ECG electrodes, respectively, that are attachable to the subject. The measurement nodes are connected to the virtual ground at the common node via short conductive paths, respectively. Each measurement node of the multiple measurement nodes includes an analog front end configured to receive and process an ECG signal from the corresponding ECG electrode; a DC power converter configured to receive a modulated optical signal with an embedded clock signal via an input fiber-optic cable, to recover DC power from the modulated optical signal, and to supply the DC power to the analog front end; and a clock recovery circuit configured to receive the modulated optical signal with the embedded clock signal via the input fiber-optic cable, to recover the embedded clock signal from the modulated optical signal, and to supply the recovered clock signal to the analog front end.

According to another representative embodiment, a measurement node is provided that is connectable to an ECG electrode attachable to a body of a subject for measuring ECG pulses from the subject. The measurement node includes an ADC configured to convert an ECG signal from the ECG electrode to a digital signal; an optical converter configured to convert the digital signal from the ADC to an optical signal, and to output the optical signal to an ECG monitor via an output fiber-optic cable; a DC power converter configured to receive a modulated optical signal from the ECG monitor with an embedded clock signal via an input fiber-optic cable, to recover DC power from the modulated optical signal, and to supply the DC power to at least the ADC and the optical converter; and a clock recovery circuit configured to receive the modulated optical signal with the embedded clock signal via the input fiber-optic cable, to recover the embedded clock signal from the modulated optical signal, and to supply the recovered clock signal to at least the ADC and the optical converter for synchronization. The ADC, the optical converter, the DC power converter, and the clock recovery circuit are connected to a virtual ground created by a common node attachable to the body of the subject via a short conductive path between the measurement node and the common node.

### BRIEF DESCRIPTION OF THE DRAWINGS

The example embodiments are best understood from the following detailed description when read with the accompanying drawing figures. It is emphasized that the various features are not necessarily drawn to scale. In fact, the dimensions may be arbitrarily increased or decreased for clarity of discussion. Wherever applicable and practical, like reference numerals refer to like elements.
FIG. 1 is a simplified block diagram showing a set of measurement nodes for monitoring electrocardiogram (ECG) signals from a subject, according to a representative embodiment.
FIG. 2 is a simplified block diagram showing an ECG system for monitoring ECG pulses from a subject, implemented within magnetic resonance imaging (MRI) system, according to a representative embodiment.
FIG. 3 is a simplified block diagram showing a representative measurement node for monitoring ECG signals from a subject, according to a representative embodiment.

### DETAILED DESCRIPTION

In the following detailed description, for the purposes of explanation and not limitation, representative embodiments disclosing specific details are set forth in order to provide a thorough understanding of an embodiment according to the present teachings. Descriptions of known systems, devices, materials, methods of operation and methods of manufacture may be omitted so as to avoid obscuring the description of the representative embodiments. Nonetheless, systems, devices, materials, and methods that are within the purview of one of ordinary skill in the art are within the scope of the present teachings and may be used in accordance with the representative embodiments. It is to be understood that the terminology used herein is for purposes of describing particular embodiments only and is not intended to be limiting. The defined terms are in addition to the technical and scientific meanings of the defined terms as commonly understood and accepted in the technical field of the present teachings.

It will be understood that, although the terms first, second, third, etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

The terminology used herein is for purposes of describing particular embodiments only and is not intended to be limiting. As used in the specification and appended claims, the singular forms of terms "a," "an" and "the" are intended to include both singular and plural forms, unless the context clearly dictates otherwise. Additionally, the terms "comprises," "comprising," and/or similar terms specify the presence of stated features, elements, and/or components, but do not preclude the presence or addition of one or more other features, elements, components, and/or groups thereof. As used herein, the term "and/or" includes any and all combinations of one or more of the associated listed items.

Unless otherwise noted, when an element or component is said to be "connected to," "coupled to," or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

The present disclosure, through one or more of its various aspects, embodiments and/or specific features or sub-components, is thus intended to bring out one or more of the advantages as specifically noted below. For purposes of explanation and not limitation, example embodiments disclosing specific details are set forth in order to provide a thorough understanding of an embodiment according to the present teachings. However, other embodiments consistent with the present disclosure that depart from specific details disclosed herein remain within the scope of the appended claims. Moreover, descriptions of well-known apparatuses and methods may be omitted so as to not obscure the description of the example embodiments. Such methods and apparatuses are within the scope of the present disclosure.

Generally, the various embodiments described herein provide an ECG system that includes synchronized measurement nodes configured to digitize and transmit ECG pulses acquired from a subject through corresponding ECG electrodes attached to a body of the subject. Each measurement node includes all necessary components at the corresponding ECG electrode to which it is attached for digitizing the ECG pulses. This eliminates the need for a conductive ECG lead to connect the measurement node to an ECG module. Without conductive ECG leads, the measurement nodes reduce signal degradation otherwise caused by noise within the bore of an MRI system, for example, caused by conventional measurement nodes and ECG modules. Each measurement node includes an analog-to-digital converter (ADC) for converting the ECG pulses to digital signals, a pair of fiber-optic cables for communicating DC power, clock and ECG pulse data with the ECG module, and a short conductive path for connecting to a common measurement node that creates a common electrical reference. The measurement nodes may be snapped or clipped onto existing ECG electrodes, or may incorporate dedicated ECG electrodes.

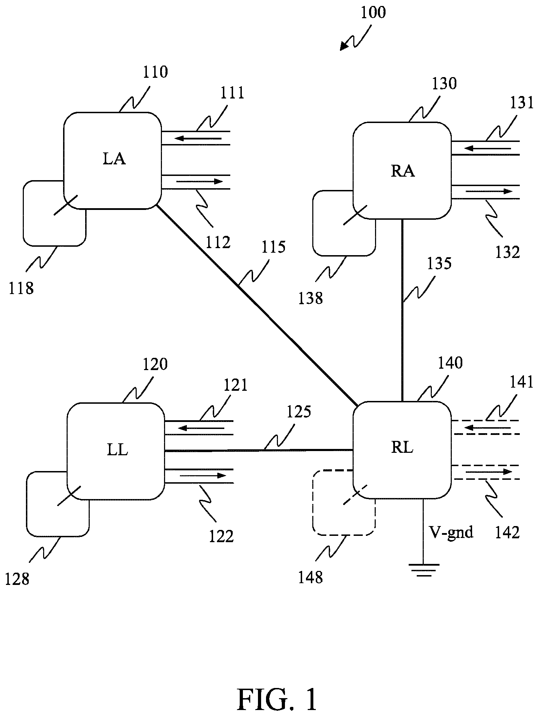

FIG. 1 is a simplified block diagram showing a set of measurement nodes for monitoring electrocardiogram (ECG) signals from a subject, according to a representative embodiment.

Referring to FIG. 1, an illustrative measurement node set 100 is attachable to the skin of a subject, e.g., using adhesive strips, for acquiring ECG pulses produced by the subject's heart beat. The measurement node set 100 includes a measurement node 110 (e.g., left arm (LA) measurement node), a measurement node 120 (e.g., left leg (LL) measurement node), a measurement node 130 (e.g., right arm (RA) measurement node), and a common node 140 (e.g., right leg (RL) measurement node). As discussed below, the measurement nodes 110, 120 and 130 include the necessary components for receiving and digitizing the ECG pulses, converting the digitized ECG pulses into optical ECG pulses, and communicating the optical pulses over optical fiber, thus eliminating the need for electrical ECG leads. In various configurations, the number of measurement nodes in the measurement node set 100 may vary (e.g., up to 12 total) to provide unique benefits for any particular situation or to meet application specific design requirements of various implementations, as would be apparent to one skilled in the art.

The common node 140 creates a common electrical reference for the measurement node set 100, referred to as virtual ground (V-gnd). In an embodiment, the common node 140 may also be a measurement node with the same configuration as the measurement nodes 110, 120 and 130. In this case, the common node 140 may also be used for receiving and digitizing the ECG pulses, converting the digitized ECG pulses into optical ECG pulses, and communicating the optical pulses over optical fiber. Alternatively, the common node 140 may simply create the virtual ground without the functionality of a measurement node.

Since the common node 140 creates the virtual ground, the measurement nodes 110, 120 and 130 are connected to the common node 140 through short conductive paths in relation to the subject's body length, so that they have a common ground potential without having to be electrically grounded, e.g., through long ECG leads. A short conductive path may be less than a quarter of the subject's body length, while the long ECG leads exceed the subject's body length. In particular, the measurement node 110 is connected to the common node 140 via conductive path 115, the measurement node 120 is connected to the common node 140 via conductive path 125, and the measurement node 130 is connected to the common node 140 via conductive path 135. Each of the short conductive paths 115, 125 and 135 is formed of a highly electrically conductive material, such as copper, aluminum, gold or silver, for example, and is no longer than about 12 cm, for example.

In the depicted embodiment, each of measurement nodes 110, 120 and 130 is connected to a corresponding ECG electrode that attaches to the skin of the subject at specific locations on the subject's body to acquire ECG pulses generated from the subject's heartbeat. In particular, the measurement node 110 is connected to ECG electrode 118, the measurement node 120 is connected to ECG electrode 128, and the measurement node 130 is connected to ECG electrode 138. The common node 140 is shown as optionally connected to ECG electrode 148 (indicated by dashed lines), which would occur when the common node 140 also has the functionality of a measurement node, as discussed above. The measurement nodes 110, 120 and 130 may be detachably connected to the ECG electrodes 118, 128 and 138, in which case conventional ECG electrodes may be used. For example, the measurement nodes 110, 120 and 130 may snap or clip onto respective upper surfaces (facing away from the subject's body) of the corresponding ECG electrodes 118, 128 and 138. Alternatively, the ECG electrodes 118, 128 and 138 may be physically integrated within the measurement nodes 110, 120 and 130, respectively.

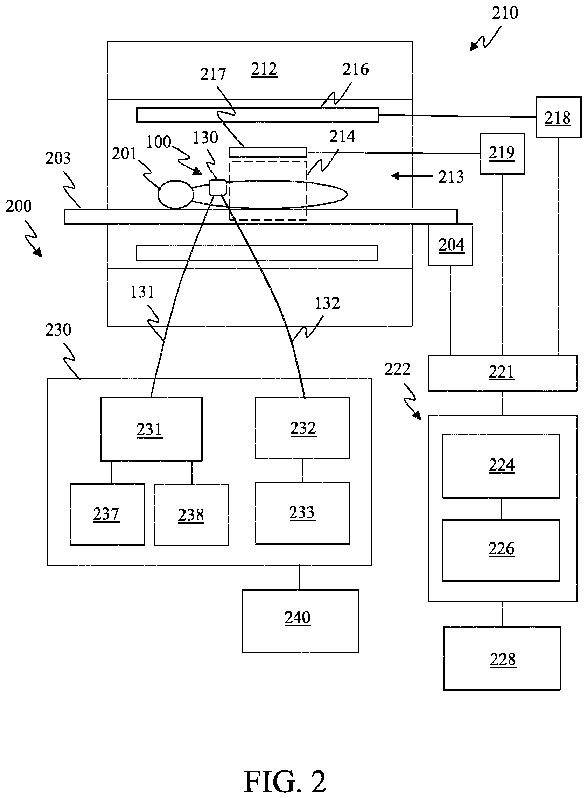

The measurement nodes 110, 120 and 130 are further configured to communicate with an ECG module (not shown), discussed below with reference to FIG. 2. Generally, the ECG module provides DC power and clock signals to the measurement nodes 110, 120 and 130 via input fiber-optic cables, and processes the ECG signals provided by the measurement nodes 110, 120 and 130 via output fiber-optic cables. In particular, the measurement node 110 is connected to input fiber-optic cable 111 and output fiber-optic cable 112, the measurement node 120 is connected to input fiber-optic cable 121 and output fiber-optic cable 122, and the measurement node 130 is connected to input fiber-optic cable 131 and output fiber-optic cable 132. The common node 140 is shown as optionally connected to input fiber-optic cable 141 and output fiber-optic cable 142 (indicated by dashed lines). As mentioned above, this because the common node 140 may be configured as a measurement node to acquire ECG signals.

FIG. 2 shows an ECG system for monitoring ECG pulses from a subject, implemented within magnetic resonance imaging (MRI) system, according to a representative embodiment. Although depicted with the MRI system for purposes of explanation, it is understood that the ECG system may be implemented on its own or with any other type of medical imaging or medical testing system, without departing from the scope of the present teachings.

Referring to FIG. 2, ECG system 200 is incorporated with representative MRI system 210 in order to monitor ECG pulses of a subject 201 during an MRI procedure. The MRI system 210 may be any type of MRI system, and the following description of the MRI system 210 is intended to be illustrative and not limiting. In the depicted example, the MRI system 210 includes a magnet 212 with a bore 213. The magnet 212 may be a superconducting cylindrical magnet, for example, although use of different types of magnets is possible, such as a split cylindrical magnet and an open magnet. An imaging zone 214 is provided in the bore 213 where the magnetic field generated by operation of the magnet 212 is strong and uniform enough to perform the magnetic resonance imaging.

The subject 201 is placed on a support 203 and positioned within the bore 213 to be imaged during the MRI procedure. The support 203 may be attached to an actuator 204 (optional) configured to move the support 203, so that the subject 201 may be moved through the imaging zone 214. Accordingly, a larger portion of the subject 201 or the entire subject 201 may be imaged.

The ECG system 200 includes the measurement node set 100, discussed above. Accordingly, the ECG electrodes 118, 128 and 138 respectively corresponding to the measurement nodes 110, 120 and 130 are attached to the skin of the subject 201 in order to perform ECG monitoring during the MRI procedure. Only the measurement node 130 is shown in FIG. 2 for the sake of convenience. As discussed above, the common node 140 (not shown) creates a virtual ground, and the measurement node 130 is connected to the common node 140 by the short conductive path 135 in order to provide the common electrical reference to the measurement node 130. The other measurement nodes 110 and 120 (not shown) are likewise connected to the virtual ground provided by the common node 140, as discussed above. In an embodiment, the common node 140 is also a measurement node, and is connected to the corresponding ECG electrode 148.

The MRI system 210 includes a set of magnetic field gradient coils 216 configured to acquire magnetic resonance data for spatially encoding magnetic spins within the imaging zone 214. A magnetic field gradient coil power supply 218 supplies current to the magnetic field gradient coils 216. The current may be controlled as a function of time, and may be ramped or pulsed, for example. Although two magnetic field gradient coils 216 are shown, it is understood that additional magnetic field gradient coils may be included, e.g., to enable spatially encoding in three orthogonal spatial directions.

The MRI system 210 further includes RF coil 217 located within the bore 213. The RF coil 217 is configured to manipulate orientations of magnetic spins within the imaging zone 214, and to receive RF transmissions from spins also within the imaging zone 214. The RF coil 217 may represent dedicated transmit and receive antennas or may contain multiple transmit and receive coil elements. The RF coil 217 is shown connected to an RF transceiver 219, which transmits and receives RF signals to and from the RF coil 217 during the MRI procedure. In various configurations, the RF coil 217 and the RF transceiver 219 may be replaced by separate transmit and receive coils and separate transmitters and receivers, for example.

The actuator 204, the magnetic field gradient coil power supply 218, and the RF transceiver 219 are connected to a hardware interface 221 and a controller 222. The controller 222 includes a processor 224, memory 226, and a user interface 228. The memory 226 represents one or more non-transitory memories and/or data storage, discussed further below. The memory 226 may store pulse sequence instructions, which are executed by the processor 224 for performing the MRI procedure. The memory 226 may also include data storage for storing magnetic resonance data and/or reconstructed magnetic resonance images acquired during the MRI procedure. The hardware interface 221 enables the controller 222 to interact with, control and/or exchange data with at least the actuator 204, the magnetic field gradient coil power supply 218, and the RF transceiver 219. The hardware interface 221 may include one or more of a universal serial bus (USB), IEEE 1394 port, parallel port, IEEE 1284 port, serial port, RS-232 port, IEEE-488 port, Bluetooth connection, wireless local area network connection, TCP/IP connection, Ethernet connection, control voltage interface, MIDI interface, analog input interface, and digital input interface, for example.

The processor 224 is representative of one or more processing devices and may be implemented by a general-purpose computer, a central processing unit, a computer processor, a microprocessor, a microcontroller, field programmable gate arrays (FPGAs), application specific integrated circuits (ASICs), a state machine, programmable logic device, or combinations thereof, using any combination of hardware, software, firmware, hard-wired logic circuits, or combinations thereof. A processor may also refer to a collection of processors within a single computer system or distributed among multiple computer systems, such as in a cloud-based or other multi-site application.

The memory 226 may be implemented by any number, type and combination of random-access memory (RAM) and read-only memory (ROM), for example, and may store various types of information, such as software algorithms, artificial intelligence (AI) machine learning models, and computer programs, all of which are executable by the processor 224. The various types of ROM and RAM may include any number, type and combination of non-transitory computer readable storage media, such as a disk drive, flash memory, an electrically programmable read-only memory (EPROM), an electrically erasable and programmable read-only memory

(EEPROM), registers, a hard disk, a removable disk, tape, compact disk read only memory (CD-ROM), digital versatile disk (DVD), floppy disk, Blu-ray disk, a universal serial bus (USB) drive, or any other form of storage medium known in the art. As used herein, the term non-transitory is to be interpreted not as an eternal characteristic of a state, but as a characteristic of a state that will last for a period. The term non-transitory specifically disavows fleeting characteristics such as characteristics of a carrier wave or signal or other forms that exist only transitorily in any place at any time.

The user interface 228 enables a user or operator to interact with the controller 222, receiving input from the operator to be received by the processor 224 and providing output to the user from the processor 224. That is, the user interface 228 may provide information or data to the operator and/or receive information or data from the operator. The display of data or information on a display or a graphical user interface is an example of providing information to the operator. The receiving of data through a keyboard, mouse, trackball, touchpad, pointing stick, graphics tablet, joystick, gamepad, webcam, headset, pedals, wired glove, remote control, and accelerometer are all examples of components of the user interface 228 which enable the receiving of information or data from the operator.

In addition to the measurement node set 100, the ECG system 200 further includes an ECG module 230 and an output 240. In the depicted embodiment, the ECG module 230 includes an optical modulator 231, an optical demodulator 232, and a processor 233. The optical modulator 231 is configured to receive a clock signal from a clock 237 and a light signal from a light source 238, to modulate the light signal and the clock signal using any compatible modulation technique, and to output a modulated optical signal with an embedded clock signal to the measurement nodes 110, 120 and 130 via the respective input fiber-optic cables 111, 121 and 131, respectively. The light source 238 may be a laser or a light emitting diode (LED), for example. In an embodiment, the optical modulator 231 may provide a pulse width modulated (PWM) optical signal with an embedded clock signal, which may be embedded via light pulses, for example. Alternatively, the optical modulator 231 may provide a frequency modulated or amplitude modulated optical signal with the embedded clock signal. The frequencies and/or widths of the light pulses in the PWM optical signal and the embedded clock signal, for example, may be adjusted to suit the MRI scanning environment. For example, certain frequencies must be avoided as to not interfere with the MR scanned image. A tunable configuration of the ECG module 230 allows all frequencies to be selected or avoided.

The optical demodulator 232 is configured to receive optical ECG signals from the measurement nodes 110, 120 and 130 via the respective output fiber-optic cables 112, 122 and 132, respectively, and to convert the ECG signals into corresponding electrical signals. The processor 233 is configured to execute instructions stored in a non-transitory memory (not shown) for processing the electrical signals to provide a corresponding ECG wave to the output 240. The instructions may further cause the processor 233 to define characteristics of the ECG signals, such as the QRS complex, average beat, heart rate variability, RR interval, PR interval, and pulse rate, for example. The memory may be one or more non-transitory memories and/or data storage, as described above with reference to the memory 226.

The processor 233 is representative of one or more processing devices, and may be implemented by a general-purpose computer, a central processing unit, a computer processor, a microprocessor, a microcontroller, FPGAs, ASICs, a state machine, programmable logic device, or combinations thereof, using any combination of hardware, software, firmware, hard-wired logic circuits, or combinations thereof. A processor may also refer to a collection of processors within a single computer system or distributed among multiple computer systems, such as in a cloud-based or other multi-site application.

The output 240 may include any type of visual manifestation of the ECG traces. For example, the output 240 may include a display for displaying the ECG wave, such as a computer monitor, a television, a liquid crystal display (LCD), an organic light emitting diode (OLED) display, a flat panel display, a solid-state display, or a cathode ray tube (CRT) display, a touch screen or an electronic whiteboard, for example. Alternatively, or in addition, the output 240 may include a printer, such as a thermal printer or an inkjet printer, for example, for printing the ECG wave. ECG wave may be displayed and/or printed together with textual and/or graphical information that classifies and/or interprets the ECG wave.

In the depicted embodiment, the measurement nodes 110, 120 and 130 are physically connected to the ECG module 230 via the input fiber-optic cables 111, 121 and 131 and the output fiber-optic cables 112, 122 and 132, respectively. However, in an alternative embodiment, the measurement nodes 110, 120 and 130 may be connected to a transceiver and antenna (not shown) via the input fiber-optic cables 111, 121 and 131 and the output fiber-optic cables 112, 122 and 132, respectively, where the transceiver is configured to communicate wirelessly with the ECG module 230. In this case, the ECG module 230 would likewise include a transceiver and antenna (not shown) for sending the DC power and clock signals and receiving the ECG signals.

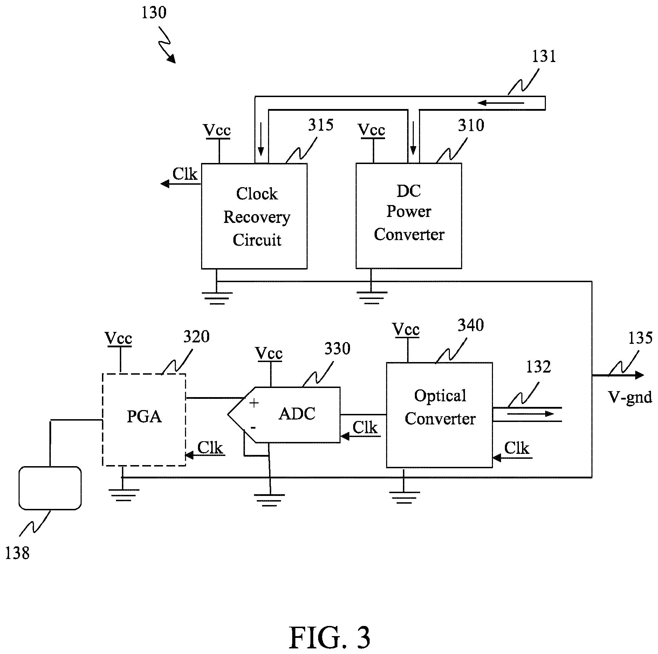

As discussed above, the measurement nodes 110, 120 and 130 have the same configuration. In various implementations, the common node 140 may also have the same configuration as the measurement nodes 110, 120 and 130 (except for the conductive paths). FIG. 3 is a simplified block diagram showing an illustrative measurement node for monitoring ECG signals from a subject, according to a representative embodiment. In particular, FIG. 3 shows the measurement node 130 as being representative of all the measurement nodes, for purposes of illustration.

Referring to FIG. 3, the measurement node 130 includes a DC power converter 310 and a clock recovery circuit 315, which are connected to the input fiber-optic cable 131. The DC power converter 310 is configured to receive the modulated optical signal from the optical modulator 231 of the ECG module 230 via the input fiber-optic cable 131, and to convert the modulated optical signal to a corresponding electrical signal. By converting the modulated optical signal to the electrical signal, the DC power converter 310 recovers DC power embedded within the modulated optical signal. For example, when the modulated optical signal is a PWM optical signal, the magnitude of the DC power is indicated by the frequency and/or widths of the light pulses. The DC power converter 310 may be a photovoltaic cell, for example, which converts optical signals directly into electrical signals using photovoltaic effect. The clock recovery circuit 315 recovers the embedded clock signal from the modulated optical signal. The clock recovery circuit 315 may be an edge detector, phase detector or a frequency detector, for example. The detectors of the clock recovery circuit depend on how the clock is optically encoded, as is known in the art. Recovery of the DC power and the embedded clock signal may be performed in any order or simultaneously. The DC power converter 310 outputs the DC power (Vcc) and the clock recovery circuit 315 outputs the recovered clock signal (Clk) to other components of the measurement node 130, discussed below.

The measurement node 130 is shown connected to the ECG electrode 138, which is attached to the skin of the subject 201, to receive small analog ECG pulses, which may be in the µV to mV ranges. The measurement node 130 provides an analog front end for the ECG electrode 138, including an optional programmable gain amplifier (PGA) 320 (indicated by dashed lines) and an analog-to-digital converter (ADC) 330, as well as an optical converter 340. As shown, each of the PGA 320, the ADC 330, and the optical converter 340 receive the DC power (Vcc) from the DC power converter 310 and the recovered clock signal (Clk) from the clock recovery circuit 315. Accordingly, the PGA 320, the ADC 330, and the optical converter 340 are powered without an electrical power source using the DC power (Vcc) and are synchronized with one another using the recovered clock signal (Clk).

The PGA 320 receives the analog ECG pulses from the ECG electrode 138, which are electrical signals. The ADC 330 converts the ECG pulses into digital ECG signals at a predetermined sampling frequency (e.g., 300 Hz). The optical converter 340 receives the digital ECG signals and converts them to optical ECG signals. The optical converter 340 may be a laser or an LED light source, for example. The optical converter 340 outputs the optical ECG signals to the optical demodulator 232 of the ECG module 230 via the output fiber-optic cable 132.

In addition, the grounds of each of the DC power converter 310, the clock recovery circuit 315, the PGA 320, the ADC 330, and the optical converter 340 are connected to the virtual ground (V-gnd) created by the common node 140 via the conductive path 135. Accordingly, the DC power and clock recovery circuit 315, the PGA 320, the ADC 330, and the optical converter 340 are grounded to a common potential, along with the components of the other measurement nodes (e.g., measurement nodes 110, 120), without having to be electrically grounded elsewhere in the ECG system 200. The recovered DC power and the virtual grounding of the measurement node 130 eliminate the need for electrical leads connecting the measurement node 130 to an external power source and ground.

The illustrations of the embodiments described herein are intended to provide a general understanding of the structure of the various embodiments. The illustrations are not intended to serve as a complete description of all of the elements and features of the disclosure described herein. Many other embodiments may be apparent to those of skill in the art upon reviewing the disclosure. Other embodiments may be utilized and derived from the disclosure, such that structural and logical substitutions and changes may be made without departing from the scope of the disclosure. Additionally, the illustrations are merely representational and may not be drawn to scale. Certain proportions within the illustrations may be exaggerated, while other proportions may be minimized. Accordingly, the disclosure and the figures are to be regarded as illustrative rather than restrictive.

Although specific embodiments have been illustrated and described herein, it should be appreciated that any subsequent arrangement designed to achieve the same or similar purpose may be substituted for the specific embodiments shown. This disclosure is intended to cover any and all subsequent adaptations or variations of various embodiments. Combinations of the above embodiments, and other embodiments not specifically described herein, will be apparent to those of skill in the art upon reviewing the description.

## Claims

1. A system (100) for acquiring electrocardiogram (ECG) pulses from a subject, the system comprising:
a common node (140) configured to create a virtual ground (V-gnd); and
a plurality of measurement nodes (110, 120, 130) connectable to a plurality of corresponding ECG electrodes (118, 128, 138), respectively, attachable to the subject;
wherein the plurality of measurement nodes are connected to the virtual ground at the common node via short conductive paths (115, 125, 135), respectively;
wherein each measurement node of the plurality of measurement nodes comprises:
an analog-to-digital converter (ADC, 330) configured to convert an ECG signal from the corresponding ECG electrode to a digital signal;
an optical converter (340) configured to convert the digital signal from the ADC to an optical signal, and to output the optical signal via an output fiber-optic cable (112, 122, 132);
a DC power converter (310) configured to receive a modulated optical signal with an embedded clock signal via an input fiber-optic cable (111, 121, 131), to recover DC power from the modulated optical signal, and to supply the DC power to at least the ADC and the optical converter; and
a clock recovery circuit (315) configured to receive the modulated optical signal with the embedded clock signal via the input fiber-optic cable, to recover the embedded clock signal from the modulated optical signal, and to supply the recovered clock signal to at least the ADC and the optical converter for synchronization.

2. The system of claim 1, further comprising:
an ECG module (230) configured to provide the modulated optical signal to the plurality of measurement nodes via the input fiber-optic cables respectively, to receive the optical signals from the plurality of measurement nodes via the output fiber-optic cables respectively, and to convert the optical signals to the ECG pulses.

3. The system of any one of claims 1 or 2, wherein the common node is configured as a measurement node connectable to a corresponding ECG electrode attachable to the subject.

4. The system of claim 3, wherein the plurality of measurement nodes comprise a left arm (LA) measurement node, a right arm (RA) measurement node, and a left leg (LL) measurement node; and
wherein the common node comprises a right leg (RL) measurement node.

5. The system of any one of claims 1-4, wherein the DC power converter of each measurement node of the plurality of measurement nodes comprises a photovoltaic cell.

6. The system of claim any one of claims 1-5, wherein each measurement node of the plurality of measurement nodes further comprises:
a programmable gain amplifier (PGA, 320) connected to an input of the ADC, and configured to amplify the ECG signal.

7. The system of any one of claims 2-6, further comprising:
a monitor configured to display the ECG pulses output by the ECG module.

8. The system of any one of claims 1-7, wherein the subject is positioned within a magnet resonance imaging (MRI) bore while the ECG pulses are acquired.

9. The system of claim any one of claims 1-8, wherein the modulated optical signal comprises to a pulse width modulated (PWM) optical signal.

10. The system of any one of claims 1-9, wherein the modulated optical signal comprises a frequency modulated or amplitude modulated optical signal.

11. The system of any one of claims 1-10, wherein each measurement node of the plurality of measurement nodes comprises:
an analog front end comprising said analog-to-digital converter (ADC) of the corresponding measurement node and configured to receive and process an ECG signal from the corresponding ECG electrode;
wherein the DC power converter is configured to supply the DC power to the analog front end; and
wherein the clock recovery circuit is configured to supply the recovered clock signal to the analog front end.

12. The system of any one of claims 1-11, wherein each measurement node of the plurality of measurement nodes is configured to snap or clip onto an upper surface of the corresponding ECG electrode.

13. The system of any one of claims 1-11, wherein each measurement node of the plurality of measurement nodes further comprises the corresponding ECG electrode physically integrated within the measurement node.

14. A measurement node (110, 120, 130) connectable to an electrocardiogram (ECG) electrode (118, 128, 138) attachable to a body of a subject for measuring ECG pulses from the subject, the measurement node comprising:
an analog-to-digital converter (ADC,330) configured to convert an ECG signal from the ECG electrode to a digital signal;
an optical converter (340) configured to convert the digital signal from the ADC to an optical signal, and to output the optical signal to an ECG monitor via an output fiber-optic cable (112, 122, 132);
a DC power converter (310) configured to receive a modulated optical signal from the ECG monitor with an embedded clock signal via an input fiber-optic cable (111, 121, 131), to recover DC power from the modulated optical signal, and to supply the DC power to at least the ADC and the optical converter; and
a clock recovery circuit (315) configured to receive the modulated optical signal with the embedded clock signal via the input fiber-optic cable, to recover the embedded clock signal from the modulated optical signal, and to supply the recovered clock signal to at least the ADC and the optical converter for synchronization,
wherein the ADC, the optical converter, the DC power converter, and the clock recovery circuit are connected to a virtual ground (V-gnd) created by a common node (140) attachable to the body of the subject via a short conductive path (115, 125, 135) between the measurement node and the common node.

## Patentansprüche

1. System (100) zum Erfassen von Elektrokardiogramm (EKG)-Impulsen von einer Person, wobei das System umfasst:
einen gemeinsamen Knoten (140), der so konfiguriert ist, dass er eine virtuelle Masse (V-gnd) erzeugt; und
eine Vielzahl von Messknoten (110, 120, 130), die mit einer Vielzahl von entsprechenden EKG-Elektroden (118, 128, 138) verbunden werden können, die jeweils an der Person angebracht werden können;
wobei die Vielzahl von Messknoten mit der virtuellen Masse am gemeinsamen Knoten über jeweils kurze leitende Pfade (115, 125, 135) verbunden sind;
wobei jeder Messknoten der Vielzahl von Messknoten umfasst:
einen Analog-Digital-Wandler (ADC, 330), der so konfiguriert ist, dass er ein EKG-Signal von der entsprechenden EKG-Elektrode in ein digitales Signal umwandelt;
einen optischen Wandler (340), der so konfiguriert ist, dass er das digitale Signal vom ADC in ein optisches Signal umwandelt und das optische Signal über einen Ausgangslichtwellenleiter (112, 122, 132) ausgibt;
einen Gleichstromwandler (310), der so konfiguriert ist, dass er ein moduliertes optisches Signal mit einem eingebetteten Taktsignal über einen Eingangslichtwellenleiter (111, 121, 131) empfängt, um Gleichstrom aus dem modulierten optischen Signal zu gewinnen und den Gleichstrom mindestens dem ADC und dem optischen Wandler zuzuführen; und
eine Taktrückgewinnungsschaltung (315), die so konfiguriert ist, dass sie das modulierte optische Signal mit dem eingebetteten Taktsignal über den Eingangslichtwellenleiter empfängt, das eingebettete Taktsignal aus dem modulierten optischen Signal zurückgewinnt und das zurückgewonnene Taktsignal mindestens dem ADC und dem optischen Wandler zur Synchronisation zuführt.

2. System nach Anspruch 1, weiter umfassend:
ein EKG-Modul (230), das so konfiguriert ist, dass es das modulierte optische Signal jeweils über die Eingangslichtwellenleiter an die Vielzahl von Messknoten liefert, die optischen Signale jeweils über die Ausgangslichtwellenleiter von der Vielzahl von Messknoten empfängt und die optischen Signale in die EKG-Impulse umwandelt.

3. System nach einem der Ansprüche 1 oder 2, wobei der gemeinsame Knoten als Messknoten konfiguriert ist, der mit einer entsprechenden EKG-Elektrode verbunden werden kann, die an der Person angebracht werden kann.

4. System nach Anspruch 3, wobei die Vielzahl von Messknoten einen Messknoten für den linken Arm (LA), einen Messknoten für den rechten Arm (RA) und einen Messknoten für das linke Bein (LL) umfasst; und
wobei der gemeinsame Knoten einen Messknoten für das rechte Bein (RL) umfasst.

5. System nach einem der Ansprüche 1-4, wobei der Gleichstromwandler jedes Messknotens der Vielzahl von Messknoten eine Photovoltaikzelle umfasst.

6. System nach einem der Ansprüche 1-5, wobei jeder Messknoten der Vielzahl von Messknoten weiter umfasst:
einen programmierbaren Verstärker (PGA, 320), der mit einem Eingang des ADC verbunden und so konfiguriert ist, dass er das EKG-Signal verstärkt.

7. System nach einem der Ansprüche 2-6, weiter umfassend:
einen Monitor, der so konfiguriert ist, dass er die von dem EKG-Modul ausgegebenen EKG-Impulse anzeigt.

8. System nach einem der Ansprüche 1-7, wobei die Person in einer Magnetresonanztomographie (MRI)-Röhre positioniert ist, während die EKG-Impulse erfasst werden.

9. System nach einem der Ansprüche 1-8, wobei das modulierte optische Signal ein pulsbreitenmoduliertes (PWM) optisches Signal umfasst.

10. System nach einem der Ansprüche 1-9, wobei das modulierte optische Signal ein frequenzmoduliertes oder amplitudenmoduliertes optisches Signal umfasst.

11. System nach einem der Ansprüche 1-10, wobei jeder Messknoten der Vielzahl von Messknoten umfasst:
ein analoges Frontend, das den Analog-Digital-Wandler (ADC) des entsprechenden Messknotens umfasst und so konfiguriert ist, dass es ein EKG-Signal von der entsprechenden EKG-Elektrode empfängt und verarbeitet;
wobei der Gleichstromwandler so konfiguriert ist, dass er den Gleichstrom dem analogen Frontend zuführt; und
wobei die Taktrückgewinnungsschaltung so konfiguriert ist, dass sie das zurückgewonnene Taktsignal dem analogen Frontend zuführt.

12. System nach einem der Ansprüche 1-11, wobei jeder Messknoten der Vielzahl von Messknoten so konfiguriert ist, dass er an einer oberen Fläche der entsprechenden EKG-Elektrode einrastet oder anklemmt.

13. System nach einem der Ansprüche 1-11, wobei jeder Messknoten der Vielzahl von Messknoten weiter die entsprechende EKG-Elektrode umfasst, die physisch in den Messknoten integriert ist.

14. Messknoten (110, 120, 130), der mit einer Elektrokardiogramm (EKG)-Elektrode (118, 128, 138) verbunden werden kann, die an einem Körper einer Person angebracht werden kann, um EKG-Impulse von der Person zu messen, wobei der Messknoten umfasst:
einen Analog-Digital-Wandler (ADC, 330), der so konfiguriert ist, dass er ein EKG-Signal von der EKG-Elektrode in ein digitales Signal umwandelt;
einen optischen Wandler (340), der so konfiguriert ist, dass er das digitale Signal vom ADC in ein optisches Signal umwandelt und das optische Signal über einen Ausgangslichtwellenleiter (112, 122, 132) an einen EKG-Monitor ausgibt;
einen Gleichstromwandler (310), der so konfiguriert ist, dass er ein moduliertes optisches Signal vom EKG-Monitor mit einem eingebetteten Taktsignal über einen Eingangslichtwellenleiter (111, 121, 131) empfängt, um Gleichstrom aus dem modulierten optischen Signal zu gewinnen und den Gleichstrom mindestens dem ADC und dem optischen Wandler zuzuführen; und
eine Taktrückgewinnungsschaltung (315), die so konfiguriert ist, dass sie das modulierte optische Signal mit dem eingebetteten Taktsignal über den Eingangslichtwellenleiter empfängt, das eingebettete Taktsignal aus dem modulierten optischen Signal zurückgewinnt und das zurückgewonnene Taktsignal mindestens dem ADC und dem optischen Wandler zur Synchronisation zuführt,
wobei der ADC, der optische Wandler, der Gleichstromleistungswandler und die Taktrückgewinnungsschaltung mit einer virtuellen Masse (V-gnd) verbunden sind, die durch einen gemeinsamen Knoten (140) erzeugt wird, der über einen kurzen leitenden Pfad (115, 125, 135) zwischen dem Messknoten und dem gemeinsamen Knoten am Körper der Person angebracht werden kann.

## Revendications

1. Système (100) d'obtention d'impulsions d'électrocardiogramme (ECG) d'un sujet, le système comprenant :
un nœud commun (140) configuré pour créer une masse virtuelle (V-gnd) ; et
une pluralité de nœuds de mesure (110, 120, 130) pouvant être connectés, respectivement, à une pluralité d'électrodes ECG (118, 128, 138) correspondantes pouvant être fixées au sujet ;
dans lequel la pluralité de nœuds de mesure sont connectés à la masse virtuelle au niveau du nœud commun par l'intermédiaire, respectivement, de trajets conducteurs courts (115, 125, 135) ;
dans lequel chaque nœud de mesure de la pluralité de nœuds de mesure comprend :
un convertisseur analogique-numérique (CAN, 330) configuré pour convertir un signal ECG de l'électrode ECG correspondante en un signal numérique ;
un convertisseur optique (340) configuré pour convertir le signal numérique du CAN en un signal optique, et pour délivrer le signal optique par l'intermédiaire d'un câble à fibre optique de sortie (112, 122, 132) ;
un convertisseur de puissance CC (310) configuré pour recevoir un signal optique modulé avec un signal d'horloge embarqué par l'intermédiaire d'un câble à fibre optique d'entrée (111, 121, 131) pour récupérer la puissance CC du signal optique modulé et pour fournir la puissance CC au moins au CAN et au convertisseur optique ; et
un circuit de récupération d'horloge (315) configuré pour recevoir le signal optique modulé avec le signal d'horloge embarqué par l'intermédiaire du câble à fibre optique d'entrée pour récupérer le signal d'horloge embarqué à partir du signal optique modulé et pour fournir le signal d'horloge récupéré au moins au CAN et au convertisseur optique pour la synchronisation.

2. Système selon la revendication 1, comprenant en outre :
un module ECG (230) configuré pour fournir le signal optique modulé à la pluralité de nœuds de mesure par l'intermédiaire, respectivement, des câbles à fibres optiques d'entrée pour recevoir les signaux optiques de la pluralité de nœuds de mesure par l'intermédiaire, respectivement, des câbles à fibres optiques de sortie et pour convertir les signaux optiques en impulsions ECG.

3. Système selon l'une quelconque des revendications 1 ou 2, dans lequel le nœud commun est configuré comme un nœud de mesure pouvant être connecté à une électrode ECG correspondante pouvant être fixée au sujet.

4. Système selon la revendication 3, dans lequel la pluralité de nœuds de mesure comprend un nœud de mesure du bras gauche (LA), un nœud de mesure du bras droit (RA) et un nœud de mesure de la jambe gauche (LL) ; et
dans lequel le nœud commun comprend un nœud de mesure de la jambe droite (RL).

5. Système selon l'une quelconque des revendications 1-4, dans lequel le convertisseur de puissance CC de chaque nœud de mesure de la pluralité de nœuds de mesure comprend une cellule photovoltaïque.

6. Système selon l'une quelconque des revendications 1-5, dans lequel chaque nœud de mesure de la pluralité de nœuds de mesure comprend en outre :
un amplificateur à gain programmable (AGP, 320) connecté à une entrée du CAN et configuré pour amplifier le signal ECG.

7. Système selon l'une quelconque des revendications 2-6, comprenant en outre :
un moniteur configuré pour afficher les impulsions ECG émises par le module ECG.

8. Système selon l'une quelconque des revendications 1-7, dans lequel le sujet est positionné dans un alésage d'imagerie par résonance magnétique (IRM) pendant que les impulsions ECG sont obtenues.

9. Système selon l'une quelconque des revendications 1-8, dans lequel le signal optique modulé comprend un signal optique modulé en largeur d'impulsion (PWM).

10. Système selon l'une quelconque des revendications 1-9, dans lequel le signal optique modulé comprend un signal optique modulé en fréquence ou modulé en amplitude.

11. Système selon l'une quelconque des revendications 1-10, dans lequel chaque nœud de mesure de la pluralité de nœuds de mesure comprend :
une extrémité avant analogique comprenant ledit convertisseur analogique-numérique (CAN) du nœud de mesure correspondant et configuré pour recevoir et traiter un signal ECG de l'électrode ECG correspondante ;
dans lequel le convertisseur de puissance CC est configuré pour fournir la puissance CC à l'extrémité avant analogique ; et
dans lequel le circuit de récupération d'horloge est configuré pour fournir le signal d'horloge récupéré à l'extrémité avant analogique.

12. Système selon l'une quelconque des revendications 1-11, dans lequel chaque nœud de mesure de la pluralité de nœuds de mesure est configuré pour s'encliqueter ou se clipser sur une surface supérieure de l'électrode ECG correspondante.

13. Système selon l'une quelconque des revendications 1-11, dans lequel chaque nœud de mesure de la pluralité de nœuds de mesure comprend en outre l'électrode ECG correspondante physiquement intégrée dans le nœud de mesure.

14. Nœud de mesure (110, 120, 130) pouvant être connecté à une électrode d'électrocardiogramme (ECG) (118, 128, 138) pouvant être fixée au corps d'un sujet pour mesurer les impulsions ECG du sujet, le nœud de mesure comprenant :
un convertisseur analogique-numérique (CAN, 330) configuré pour convertir un signal ECG de l'électrode ECG en un signal numérique ;
un convertisseur optique (340) configuré pour convertir le signal numérique du CAN en un signal optique et pour délivrer le signal optique à un moniteur ECG par l'intermédiaire d'un câble à fibre optique de sortie (112, 122, 132) ;
un convertisseur de puissance CC (310) configuré pour recevoir un signal optique modulé du moniteur ECG avec un signal d'horloge embarqué par l'intermédiaire d'un câble à fibre optique d'entrée (111, 121, 131) pour récupérer la puissance CC du signal optique modulé et pour fournir la puissance CC au moins au CAN et au convertisseur optique ; et
un circuit de récupération d'horloge (315) configuré pour recevoir le signal optique modulé avec le signal d'horloge embarqué par l'intermédiaire du câble à fibre optique d'entrée pour récupérer le signal d'horloge embarqué à partir du signal optique modulé et pour fournir le signal d'horloge récupéré au moins au CAN et au convertisseur optique pour la synchronisation,
dans lequel le CAN, le convertisseur optique, le convertisseur de puissance CC et le circuit de récupération d'horloge sont connectés à une masse virtuelle (V-gnd) créée par un nœud commun (140) pouvant être fixé au corps du sujet par l'intermédiaire d'un trajet conducteur court (115, 125, 135) entre le nœud de mesure et le nœud commun.
